# EUROPÄISCHE PATENTANMELDUNG

(11) **EP 1 506 783 A1**
(43) Veröffentlichungstag der Anmeldung: **16.02.2005**
(21) Anmeldenummer: 04018056.4
(22) Anmeldetag: 30.07.2004
(51) Int. Cl.: A61K 35/78

(54) **Efeublätter zur Vergrösserung der weiblichen Brust**

(30) Priorität: 30.07.2003 DE 10335173
(71) Anmelder: Kiener, Mirjana, 4053 Basel (CH)
(72) Erfinder: Kiener, Mirjana, 4053 Basel (CH)
(74) Vertreter: Weiss, Peter, Dr. rer. nat.

(57) **Zusammenfassung**

Ein Mittel zum Vergrössern der weiblichen Brust soll aus Efeublättern oder einem Extrakt von Efeublättern oder aus Bestandteilen des Efeublatts gänzlich oder zumindest teilweise bestehen.

## Beschreibung

Die Erfindung betrifft ein Mittel zum Vergrösserung der weiblichen Brust.

### Stand der Technik

In der plastischen Chirurgie werden Vergrösserungs-Operationen des Busens durch Implantation von Silicon oder ähnlichen Verbindungen vorgenommen. Diese Operationen sind für die Patienten mit erheblichen Nachteilen verbunden. Meist ist ein stationärer Aufenthalt in einer entsprechenden Spezialklinik notwendig. Zudem bleibt in der Regel eine sichtbare Narbe übrig.

Bekannt ist auch die interne und externe Applikationen von hormonhaltigen Präparaten. Diese haben aber meist sehr unangenehme Nebenwirkungen.

Aus der WO 98/15258 ist ein nicht-chirurgisches Verfahren zur Brust vergrösserung bekannt, welches den Gebrauch von Kakaobutter und Vitamin E umfasst.

In der EP 0 281 647 wird ein Mittel zum Vergrössern der weiblichen Brust vorgeschlagen, welches aus einer Kombination folgender organischer Verbindungen besteht: Glycerin, Ölsäure, Stearinsäure, Palmitinsäure, Paraffinium perliquidum und Linolsäure.

### Aufgabe

Der vorliegenden Erfindung liegt die Aufgabe zu Grunde, ein Mittel der obengenannten Art anzugeben, welches völlig natürlich, ohne Nebenwirkungen und ohne schmerzhafte Eingriffe in den menschlichen Körper zu einer Vergrösserung der weiblichen Brust führt.

### Lösung der Aufgabe

Zur Lösung dieser Aufgabe führt, dass das Mittel aus Efeublättern oder einem Extrakt von Efeublättern oder aus synthetisch hergestellten Bestandteilen des Efeublatts gänzlich oder zumindest teilweise besteht.

Die Efeugewächse sind eine Familie mit vorwiegend tropisch-subtropischer Verbreitung; sie sind aber auch in gemässigten Klimaten vertreten. Während manche tropischen Efeu-Gewächse nur Zimmerpflanzen sind, wird beispielsweise die Hedera helix auch arzneilich genutzt. Aus toxikologischer Sicht ist das in der Familie häufige Vorkommen von Triterpensaponinen und Polyinen von Bedeutung. Als einzige einheimische Pflanze enthält auch das Efeu in allen Organen Saponine vom Triterpentyp. Die in ihrer Struktur bekannten Verbindungen, wie z. B. das Hederasaponin C, können aus den Blättern isoliert werden. Bislang findet aber das Efeublatt beziehungsweise ein Extrakt aus dem Efeublatt nur Anwendung zur Behandlung der Atemwege, insbesondere zur Behandlung von Symptomen der chronischen Bronchitis.

Gemäss der vorliegenden Erfindung sollen jedoch die Efeublättern, ein Extrakt aus den Efeublättern oder die synthetisch hergestellten Bestandteile eines Efeublatts zum Zwecke der Brustvergrösserung eingenommen werden. Dies kann gegebenenfalls zusammen mit einem Konservierungsmittel erfolgen.

Hauptwirkstoffe des erfinderischen Mittels sind ätherische Öle (frische Blätter 0,1-0,3%), Hederagenin (2,5 bis 6%), Hederasaponine (10 ml Lösung enthalten Efeublättern (33 bis 50 mg entsprechend 10 mg Hederacosid C, Flavonolglycoside.

Die empfohlene Tagesdösis beträgt 2 x 5 ml des Mittels.

Zusätzlich zu der oben beschriebenen Erfindung ist noch die positive Wirkung von Hederahelix hervorzuheben. Hederahelix besitzt antibakterielle, analgetische und sedative Effekte und eine geringe Cytotoxizität gegen verschiedene Tumorzellen.

## Patentansprüche

1. Mittel zum Vergrössern der weiblichen Brust,
**dadurch gekennzeichnet,**
**dass** es aus Efeublättern oder einem Extrakt von Efeublättern oder aus synthetisch hergestellten Bestandteilen des Efeublatts gänzlich oder zumindest teilweise besteht.

2. Mittel nach Anspruch 1, **dadurch gekennzeichnet, dass** ein Konservierungsmittel hinzugefügt ist.

3. Mittel nach Anspruch 1 oder 2, **dadurch gekennzeichnet, dass** das Mittel in flüssiger oder kapselierter Form eingenommen wird.

4. Mittel nach einem der Ansprüche 1 bis 3, **dadurch gekennzeichnet, dass** es ätherische Öle, Hederagenin, Hederasaponine, Hederacosid C und/oder Flavonolglykoside enthält.

5. Mittel nach Anspruche 4, **dadurch gekennzeichnet, dass** es 0,1 - 0,3% ätherische Öle, 2,5 - 6 % Hederagenin und Hederasaponine enthält.

6. Verwendung von Efeublätter zur Herstellung eines Mittels zur Vergrösserung der weiblichen Brust.

7. Verwendung von Efeublättern zur Herstellung eines topisch einzunehmenden Mittels zur Vergrösserung der weiblichen Brust.
